(19) Europäisches Patentamt
European Patent Office
Office européen des brevets

(11) Numéro de publication: **0 089 880 B1**

(12) **FASCICULE DE BREVET EUROPEEN**

(45) Date de publication du fascicule du brevet: **24.06.87**

(51) Int. Cl.[4]: **A 61 K 39/44**, C 12 N 11/06

(21) Numéro de dépôt: **83400523.3**

(22) Date de dépôt: **15.03.83**

(54) **Nouveaux conjugués associant, par liaison covalente, une enzyme et un anticorps, et associations médicamenteuses utilisant lesdits conjugués.**

(30) Priorité: **17.03.82 FR 8204547**

(43) Date de publication de la demande: **28.09.83 Bulletin 83/39**

(45) Mention de la délivrance du brevet: **24.06.87 Bulletin 87/26**

(84) Etats contractants désignés: **AT BE CH DE FR GB IT LI LU NL SE**

(56) Documents cités:
**BIOLOGICAL ABSTRACTS, vol. 71, 1981, page 3275, résumé no. 31289, Philadelphia, P.A., USA, T.I. BOGACHEVA et al.: "Covalent coupling of proteolytic enzymes to antibodies"**
**CHEMICAL ABSTRACTS, vol. 96, no. 5, 1er février 1982, page 469, no. 32979z, Columbus, Ohio, USA, J.R. HARPER: "Preparation and characterization of covalently linked enzyme-antibody conjugates and their use in measuring minute quantities of protein antigens"**

(73) Titulaire: **SANOFI, société anonyme, 40, Avenue George V, F-75008 Paris (FR)**

(72) Inventeur: **Jansen, Franz, Chemin des Sesquets, F-34160 Assas (FR)**
Inventeur: **Gros, Pierre, 18 rue des Muriers, F-34100 Montpellier (FR)**

(74) Mandataire: **Gillard, Marie-Louise et al, Cabinet Beau de Loménie 55, Rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 089 880 B1

**Description**

La présente invention concerne:

– des produits qui sont des conjugués associant par liaison covalente une enzyme et un anticorps ou fragments d'anticorps, dans une association médicamenteuse, avec une immunotoxine, contenant lesdits produits nouveaux.

La présente invention concerne donc:

– des conjugués immunoenzymatiques pour utilisation comme potentialisateurs d'immunotoxines dans une association médicamenteuse contenant une immunotoxine obtenue par couplage, par liaison covalente, de la chaîne A de la ricine avec des anticorps ou fragments d'anticoprs dirigés contre un antigène porté par une cellule cible, caractérisés en ce qu'ils résultent du couplage chimique par liaison covalente, entre un anticorps ou un fragment d'anticorps ayant conservé la capacité de reconnaître un antigène porté par la même cellule-cible et une enzyme capable de libérer des ions ammonium à partir de substrats naturels bien tolérés dans les organismes animaux supérieurs.

Dans la suite de ce brevet de tels composés serons désignés sous le nom de conjugués immunoenzymatiques.

Les conjugués immunoenzymatiques sont des molécules mixtes artificielles dans lesquelles l'enzyme est associée par liaison covalente à un anticorps dirigé contre un antigène porté par les cellules-cibles.

Les enzymes utilisées sont des composés connus. L'anticorps utilisé sera soit de caractère polyclonal s'il résulte d'une immunisation conventionnelle conduite chez l'animal, soit de caractère monoclonal s'il est produit par un clone de cellules hybrides obtenues par fusion entre lymphocytes et cellules de myelome. Cet anticorps pourra être utilisé, soit sous la forme de molécules entières d'immunoglobuline comportant la capacité à reconnaître l'antigène choisi, soit sous la forme de tout fragment de ces molécules d'immunoglobuline ayant conservé la capacité à reconnaître l'antigène choisi, et en particulier les fragments connus sous les appellations de $F(ab')_2$, Fab et Fab'.

Le couplage chimique entre l'anticorps (ou fragment d'anticorps) et l'enzyme peut être réalisé par de nombreuses méthodes sous réserve que la méthode choisie:

– préserve les activités biologiques respectives des deux composants du conjugué: l'anticoprs et l'enzyme;

– assure au procédé une reproductibilité satisfaisante et un bon rendement de couplage;

– permettre de maîtriser la valeur du rapport enzyme/anticorps dans le conjugué obtenu;

– conduise à un produit stable et soluble dans l'eau.

Parmi les méthodes répondant à ces caractéristiques, il faut privilégier celles qui mettent en œuvre une ou plusieurs fonctions thiol pour l'établissement de la liaison entre les deux protéines. Ces fonctions thiol peuvent appartenir indifféremment à l'une ou l'autre des protéines à coupler, ou bien être artificiellement introduites sur l'une ou l'autre de ces protéines qui ne possède pas naturellement de thiol.

Si un ou des groupements thiol doivent ainsi être artificiellement introduits sur l'une des protéines, cela pourra être réalisé par action sur cette protéine de l'anhydride S-acétylmercapto-succinique qui est capable d'acyler des fonctions aminées de la protéine. On pourra ensuite libérer les fonctions thiol par élimination du radical acétyl protecteur, par action de l'hydroxylamine, ainsi que cela a été décrit [Archives of Biochemistry 119, 41–49, (1967)]. Une dialyse permet d'éliminer les excès de réactifs ainsi que les produits de réaction de faible masse moléculaire. D'autres méthodes décrites dans la littérature peuvent aussi être utilisées pour l'introduction de fonctions thiol dans une des protéines à coupler.

Selon l'invention, celle des deux protéines qui seule possède une ou plusieurs fonctions thiol est mise en réaction avec l'autre protéine dans laquelle auront été préalablement introduites une ou plusieurs fonctions susceptibles de réagir avec les thiols, en milieu aqueux de pH compris entre 5 et 9, et à une température ne dépassant pas 30 °C, pour fournir une liaison covalente stable et définie. Cette liaison covalente sera en particulier, soit une liaison disulfure, soit une liaison thioéther. On désigne par la suite par $P_1$ celle des deux protéines qui porte la ou les fonctions thiol et par $P_2$ l'autre protéine à coupler.

1) Cas de la liaison disulfure:

La préparation du conjugué peut alors être représentée par le schéma:

$$P_1 - SH + P_2\text{–}S\text{–}S\text{–}X \longrightarrow P_1\text{–}S\text{–}S\text{–}P_2\ XSH$$

dans lequel:

–S–S–X désigne un groupement disulfure mixte activé dont X est le radical activateur.

La protéine $P_2$ substituée par un atome de soufre activé est obtenue à partir de la protéine $P_2$ elle-même, par substitution à l'aide d'un réactif lui-même porteur d'un atome de soufre activé selon le schéma:

$$P_2 + Y\text{–}R\text{–}S\text{–}S\text{–}X \longrightarrow P_2\text{–}R\text{–}S\text{–}S\text{–}X$$

dans lequel:

$P_2$ désigne la protéine à substituer

Y représente une fonction permettant la fixation covalente du réactif sur la protéine

R désigne un groupement pouvant porter simultanément les substituants Y et –S–S–X

X désigne le radical activateur.

Le groupement fonctionnel Y est une fonction capable de se lier de façon covalente avec l'une quelconque des fonctions portées par les chaînes latérales des aminoacides constitutifs de la protéine à substituer. Parmi celles-ci, les fonctions aminées terminales des radicaux lysyle contenues dans la protéine sont particulièrement indiquées. Dans ce cas, Y pourra notamment représenter:

– un groupe carboxylique qui pourra se lier aux fonctions aminées de la protéine en présence d'un agent de couplage tel qu'un carbodiimide et notamment un dérivé soluble dans l'eau comme l'éthyl-1 (diéthylamino-3 propyl)-3-carbodiimide,

– un chlorure d'acide carboxylique qui est susceptible de réagir directement avec les fonctions aminées pour les acyler,

– un ester dit «activé» tel qu'un ester d'ortho- ou para-, nitro- ou dinitro-phényle ou encore un ester de N-hydroxysuccinimide qui réagit directement avec les fonctions aminées pour les acyler,

– un anhydride interne d'un diacide carboxylique tel, par exemple, l'anhydride succinique qui réagit spontanément avec les fonctions amines pour créer des liaisons amides,

– un groupement imidoester 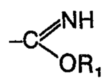

où $R_1$ est un groupe alkyle réagissant avec les groupes aminés de la protéine selon la réaction:

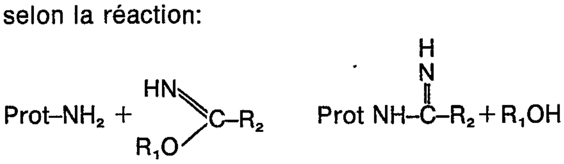

Le radical –S–S–X désigne un disulfure mixte activé capable de réagir avec un radical thiol libre. En particulier dans le disulfure mixte, X pourra désigner un groupe pyridyl-2 ou pyridyl-4 éventuellement substitué par un ou des radicaux alkyle, halogène, carboxylique. X peut aussi désigner un groupe phényle de préférence substitué par un ou des groupes nitro- ou carboxylique. X peut encore représenter un groupe alcoxycarbonyle tel que le groupe méthoxycarbonyle.

Le radical R désigne tout radical capable de porter simultanément les substituants Y et S–S–X. Il devra être choisi de façon à ne pas comporter de fonctions susceptibles d'interférer au cours des réactions ultérieures avec les réactifs utilisés et les produits synthétisés. En particulier, le groupe R peut être un groupe $-(CH_2)_n$ avec n compris entre 1 et 10 ou encore un groupe:

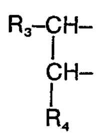

dans lequel $R_4$ désigne l'hydrogène ou un groupe alkyle ayant de 1 à 8 atomes de carbone et $R_3$ désigne un substituant inerte vis-à-vis des réactifs utilisés ultérieurement tel qu'un groupe carbamate

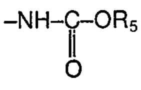

où $R_5$ désigne un groupe alkyle droit ou ramifié ayant de 1 à 5 atomes de carbone et notamment le groupe tertiobutyle.

La réaction du composé Y–R–S–S–X avec la protéine $P_2$ est effectuée en phase liquide homogène le plus souvent dans l'eau ou une solution tampon. Lorsque la solubilité des réactifs l'exige, il est possible d'ajouter au milieu réactionnel jusqu'à 20% en volume d'un solvant organique miscible à l'eau tel qu'un alcool et notamment le butanol tertiaire.

La réaction est effectuée à température ambiante pendant un temps variant de quelques heures à 24 heures. Après quoi, une dialyse permet d'éliminer les produits de faible masse moléculaire et, en particulier, les excès de réactifs. Ce procédé permet d'introduire un nombre de groupements substituants par mole de protéine habituellememnt compris entre 1 et 15.

En utilisant de tels composés, le couplage avec la protéine $P_1$ est réalisé par mise en présence en solution aqueuse des deux protéines à une température ne dépassant pas 30 °C pendant un temps variant de quelques heures à un jour. La solution aqueuse obtenue est dialysée pour éliminer les produits de faible masse moléculaire, puis le conjugué peut être purifié par diverses méthodes connues.

2) Cas de la liaison thioéther:

La préparation du conjugué consiste alors à faire réagir $P_1$ –SH avec la protéine $P_2$ sur laquelle aura préalablement été introduit un radical maléimide.

La réaction est alors représentée par le schéma:

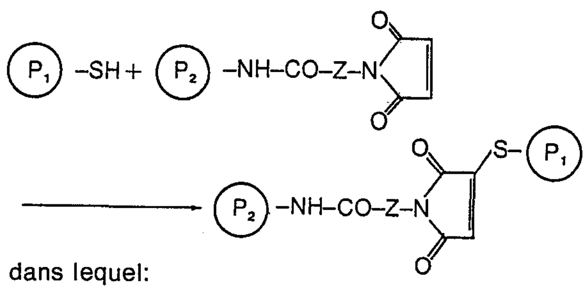

dans lequel:

Z représente une structure d'espacement, aliphatique ou aromatique comportant de 1 à 10 atomes de carbone.

La protéine $P_2$ substituée par le maléimide est obtenue à partir de la protéine $P_2$ elle-même, par substitution de fonctions aminées de la protéine à l'aide d'un réactif lui-même porteur du groupement maléimide, selon le schéma:

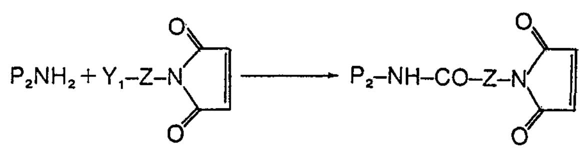

dans lequel $Y_1$ représente:

– soit un groupe carboxylique, la réaction étant alors effectuée après activation de la fonction carboxylique en présence d'un agent de couplage tel qu'un carbodiimide et notamment un dérivé soluble dans l'eau tel que l'éthyl-1 (diéthylamino-3 propyl)-3-carbodiimide,

– soit un ester dit «activé» tel qu'un ester d'ortho- ou para-, nitro- ou dinitrophényle, ou encore un ester de N-hydroxysuccinimide qui réagit directement avec les fonctions aminées pour les acyler.

La préparation de tels réactifs est notamment décrite dans Helvetica Chimica Acta 58, 531–541, (1975). D'autres réactifs de la même classe sont commercialement disponibles.

La réaction du composé $Y_1$–Z– 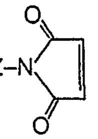

avec la protéine $P_2$ est effectuée en phase liquide homogène le plus souvent dans l'eau ou une solution tampon. Lorsque la solubilité des réactifs l'exige, il est possible d'ajouter au milieu réactionnel jusqu'à 20% en volume d'un solvant organique miscible à l'eau tel qu'un alcool et notamment le butanol tertiaire.

La réaction est effectuée à température ambiante pendant un temps variant de quelques heures à 24 heures. Après quoi, une dialyse permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine $P_1$ est réalisé par mise en présence en solution aqueuse des deux protéine à une température ne dépassant pas 30 °C pendant un temps variant de quelques heures à un jour. La solution obtenue est dialysée pour éliminer les produits de faible masse moléculaire, puis le conjugué peut être purifié par diverses méthodes connues.

De tels conjugués immunoenzymatiques peuvent être réalisés avec n'importe quelle enzyme. Toutefois, en vue de leur utilisation thérapeutique qui sera exposée ci-après, les enzymes sont celles capables de libérer des ions ammonium à partir de substrats naturels bien tolérés dans les organismes animaux supérieurs.

Selon la classification internationale telle qu'elle a été présentée par exemple dans «Comprehensive Biochemistry» tome 13, 3e édition (1973), M. Florkin et E.H. Stortz Editors (Elsevier), les enzymes utilisables en thérapeutique dans le cadre de la présente invention se retrouvent principalement:

– dans le groupe 1 (oxydoréductases) et en particulier dans le sous-groupe 1–4 contenant les aminoacide-déshydrogénases et les amine-oxydases;

– dans le groupe 3 (hydrolases) et en particulier dans le sous-groupe 3–5 contenant les enzymes hydrolysant les amides, amidines et autres liaisons C–N (à l'exclusion des liaisons peptidiques);

– dans le groupe 4 (lyases) et en particulier dans les sous-groupes 4–2 et 4–3 contenant les enzymes catalysant des réactions de dégradation avec formation de composés insaturés.

On indique ci-après la liste des enzymes préférées pour réaliser des conjugués immunoenzymatiques selon l'invention. Dans chaque cas, on a indiqué également le codage utilisé pour désigner ces enzymes dans la nomenclature internationale.

1-4-1-1 : alanine déshydrogénase
1-4-1-3 : glutamate déshydrogénase NAD $(P)^+$
1-4-1-5 : L-amino-acides déshydrogénase
1-4-3-2 : L-amino-acides oxydase
3-5-1-1 : asparaginase
3-5-1-2 : glutaminase
3-5-1-4 : amidase
3-5-1-5 : uréase
3-5-3-6 : arginine désiminase
3-5-4-4 : adénosine désaminase
3-5-4-6 : adénosine monophosphate désaminase
3-5-4-21 : créatinine désaminase
4-2-1-13 : L-sérine déshydratase
4-2-1-16 : L-théoninedéshydratase
4-3-1-1 : aspartate-ammonia-lyase (ou aspartase)
4-3-1-3 : histidine-ammonia-lyase (ou histidase)
4-3-1-5 : phénylalanine-ammonia-lyase.

Un second aspect de l'invention concerne l'utilisation en thérapeutique humaine de tels conjugués immunoenzymatiques.

Dans les demandes de brevets français antérieures, portant notamment les numéros 2 437 213, 2 466 252, 2 504 010 et 2 516 794, on a décrit la préparation de produits anticancéreux dits conjugués obtenus par couplage, par liaison covalente, de la chaîne A de la ricine avec des anticorps ou fragments d'anticorps dirigés contre un antigène porté par une cellule à détruire. Les produits de ce type sont désignés dans la présente demande sous le nom générique d'immunotoxines.

Dans la demande française 2 516 794 on a décrit en outre la propriété des ions ammonium (sous la forme d'un quelconque de leurs sels et en particulier le chlorure) de potentialiser de façon efficace l'action cytotoxique de ces immunotoxines.

La propriété des sels d'ammonium de potentialiser l'activité cytotoxique sélective des immunotoxines présente de nombreux avantages dans deux types de situations:

a) Chaque fois qu'une immunotoxine est utilisée en tant qu'agent cytotoxique sélectif in vitro pour détruire les cellules-cibles.

Dans le cas d'utilisation thérapeutique, cette situation se rencontre notamment lorsque l'immunotoxine est utilisée comme agent cytotoxique dans le traitement de la moelle osseuse de patients leucémiques à qui la moelle osseuse ainsi traitée sera ultérieurement transplantée, ainsi que cela a été décrit dans la demande de brevet déposée en France sous le n° 2 515 794 par la demanderesse

b) Lorsque l'immunotoxine est utilisée in vivo en tant qu'agent thérapeutique chez l'homme, chaque fois qu'il est possible d'administrer au malade, préalablement, simultanément ou ultérieurement à l'immunotoxine, un sel d'ammonium assurant la potentialisation de l'effet de l'immunotoxine ainsi que cela a été décrit dans la demande de brevet indiquée ci-dessus.

Toutefois, dans ce dernier cas d'utilisation, in vivo de l'immunotoxine, l'emploi, également in

vivo, d'un sel d'ammonium afin de bénéficier de l'effet potentialisateur connaît certaines limitations inhérentes à la toxicité propre des ions ammonium et au fait qu'il est relativement malaisé de maintenir de façon durable une concentration suffisante en ions ammonium dans les liquides biologiques du malade.

Les travaux poursuivis par la demanderesse ont permis de réduire de façon très importante les limitations liées à l'utilisation des ions ammonium tout en conservant les avantages de la potentialisation de l'activité cytotoxique et de l'accélération des cinotiques d'action des immunotoxines par ces ions. Ces travaux ont en effet montré que l'effet potentialisateur et accélérateur obtenu par addition aux immunotoxines d'un sel d'ammonium à la concentration convenable pouvait aussi bien être obtenu si les ions ammonium étaient produits dans l'environnement immédiat des cellules-cibles par une réaction enzymatique à partir d'un substrat non toxique naturellement présent ou artificiellement apporté dans l'environnement de ces cellules.

En outre, ces travaux ont montré que ce résultat est obtenu de façon particulièrement efficace lorsque l'enzyme qui catalyse la réaction produisant les ions ammonium est couplée à un anticorps (ou fragment d'anticorps) possédant la capacité de reconnaître un antigène présent à la surface des cellules-cibles.

Cette façon de procéder entraîne plusieurs avantages de grande importance et notamment les suivants:

a) L'enzyme utilisée est ainsi concentrée sur la membrane des cellules-cibles en raison de l'affinité de l'anticorps (ou fragment d'anticorps) pour un antigène présent sur cette membrane. De ce fait, la libération d'ions $NH_4^+$ comme produit de la réaction enzymatique n'aura lieu que dans l'environnement immédiat de la membrane des cellules-cibles, ce qui réduit les risques liés à la toxicité générale des ions ammonium, tout en facilitant l'interaction de ces ions avec la cellule-cible telle qu'elle est nécessaire pour que la potentialisation se produise.

b) La réaction enzymatique produisant les ions $NH_4^+$ d'une façon continue aussi longtemps que le substrat est présent, et ce substrat étant choisi pour être non toxique, ce procédé permet une très grande souplesse d'utilisation du mécanisme de potentialisation. En effet,

– Si le substrat est endogène et a une concentration suffisante, l'administration du conjugué immunoenzymatique potentialisateur pourra être faite avant, ou en même temps, ou après l'administration de l'immunotoxine, selon les modalités optimales à définir pour chaque patient.

– Si en outre l'enzyme est choisie de telle sorte que son substrat n'existe pas dans le sang et les liquides extracellulaires à une concentration assez élevée et que ce substrat doive donc aussi être administré au malade, la souplesse maximale d'utilisation du médicament apparaît alors puisqu'on peut régler librement et indépendamment le moment d'administration, la durée d'administration et la dose de chacun des trois composants administrés, à savoir l'immunotoxine, le conjugué immunoenzymatique et le substrat, selon les modalités optimales pour le traitement de chaque patient.

c) Dans les conditions indiquées ci-dessus, on dirigera de façon sélective vers les cellules-cibles au moins deux substances effectrices indispensables au résultat recherché:

– d'une part la chaîne A de ricine qui est l'effecteur cytotoxique inclus dans le conjugué dit immunotoxine;

– d'autre part l'enzyme qui est un constituant essentiel du système potentialisateur, inclus dans le conjugué immunoenzymatique.

Ces substances effectrices sont dans tous les cas couplées, en vue de leur pilotage et de leur fixation sélective sur les cellules-cibles, à des anticorps (ou fragments d'anticorps) reconnaissant des antigènes présents à la surface des cellules-cibles. Si l'on dispose d'un anticorps reconnaissant un antigène strictement spécifique de la population cellulaire à détruire, on peut alors utiliser le même anticorps pour le couplage aux différents effecteurs. Toutefois, dans le cas le plus général, on a intérêt à choisir des anticorps différents, reconnaissant des antigènes différents mais portés en commun par les cellules-cibles. Ainsi, même si chacun de ces antigènes n'est pas strictement spécifique des cellules-cibles, la probabilité que les deux antigènes choisis soient présents ensemble sur des cellules-non-cibles devient très faible et l'on dispose ainsi d'un moyen extrêment puissant pour augmenter encore le caractère spécifique de la cytotoxicité des immunotoxines.

Les exemples suivants illustrent l'invention sans en limiter la portée.

Exemple 1

Conjugué immunoenzymatique obtenu par réaction entre un anticorps anti-dinitrophényle substitué par un groupe disulfure activé et une uréase d'origine végétale.

a) Anticorps anti-dinitrophényle (anti-DNP)

Cet anticorps est un anticorps monoclonal qui a été purifié par des techniques conventionnelles à partir du liquide d'ascite de souris de souche Balb/C auxquelles a été transplanté l'hybridome $F_9$.

Cet hybridome a lui-même été obtenu par fusion entre des cellules spléniques de souris de souche Balb/C immunisées à l'aide de $\gamma$ globuline bovine sur laquelle ont été préalablement fixés 20 radicaux DNP par mole avec des cellules de la lignée de myélome murin NS 1, et isolé par clonage, selon les techniques classiques. L'anticorps obtenu est une immunoglobuline de classe G et d'isotype 2b dont la constante d'affinité (mesurée pour le ligand ε-DNP-lysine) est de $1,8 \cdot 10^8$ $M^+$.

b) Anticorps anti-DNP activé

Ce produit a été obtenu à partir de l'anticorps ci-dessus, selon une technique analogue à celles

décrites dans les demandes antérieures nº 7 827 838 et additif 7 924 655, nº 8 107 596 et nº 8 121 836. On a ainsi obtenu 20 mg d'anticorps anti-DNF possédant 1,2 groupement activateur par mole.

c) Uréase

L'enzyme utilisée est l'uréase de provenance SIGMA (type VII, référence U 0376), titrant 170 unités par mg. Une unité est la quantité d'enzyme qui permet la libération de 1 micromole de $NH_4^+$ par minute à 20 °C et à pH 7,0 à partir d'urée.

Cette enzyme possède naturellement 27 groupements thiol par molécule de poids moléculaire 480 000. Ces groupements thiol, titrables par la méthode d'ELLMAN, ne sont pas tous nécessaires à l'activité enzymatique. Certains peuvent donc servir à assurer le couplage avec l'anticorps activé.

d) Couplage de l'anticorps et de l'enzyme

5 mg d'uréase sont dissous dans 0,625 ml de solution d'anticorps activé à 7,2 mg/ml dans le tampon phosphate 0,125 M, pH 7,0, soit 4,5 mg d'anticorps activé et on effectue l'incubation à 25 °C pendant 14 heures.

On chromatographie le mélange réactionnel sur une colonne de gel de Sepharose 6 B® (Pharmacia) équilibrée dans un tampon PBS (phosphate 10 mM, chlorure de sodium 140 mM, pH 7,4). L'élution est suivie par mesure de la densité optique à 280 nm et par mesure de l'activité uréasse selon le technique de SUMMER [Mothode in Enzymology vol. II, p. 378, S.B. Colowick et N.O. Kaplan Ed., Academic Press, 1955].

On réunit les fractions contenant les plus fortes activités uréase, et l'on obtient ainsi 8 ml de solution de conjugué à 6,5 unités/ml. Si une fraction aliquote de cette solution est absorbée sur une colonne de sérum albumine bovine substituée par six radicaux DNP par mole et insolubilisée sur une matrice de Sepharose 4 B préalablement activé: par le bromure de cyanogène, on constate que l'activité uréase reste entièrement absorbée sur la colonne. Ceci démontre en même temps que l'anticorps présent dans le conjugué a conservé sa capacité à reconnaître l'haptène DNP et que l'uréase est bien couplée à cet anticorps.

Exemple 2

Potentialisation de l'immunotoxine anti T65

Le conjugué selon l'invention, obtenu comme indiqué précédemment, a été étudié en ce qui concerne ses propriétés biologiques et plus spécialement sa capacité à potentialiser l'activité de l'immunotoxine anti T65 dans un modèle cellulaire approprié.

Ce modèle est constitué par des cellules de la lignée lymphoblastoïde humaine CEM qui portent naturellement l'antigène T65. Cet antigène, contre lequel est dirigée l'immunotoxine utilisée, constitue le premier antigène-cible du modèle. On peut, en outre, marquer ces cellules à l'aide de l'haptène trinitrophényle (TNP) selon la technique décrite dans la demande antérieur nº 7 827 838. Cet haptène est très bien reconnu par l'anticorps anti-DNP contenu dans le conjugué immunoenzymatique étudié et constitue donc le deuxième antigène-cible du modèle. Il a été démontré que le marquage des cellules par l'haptène TNP ne modifie pas la viabilité des cellules et ne perturbe pas la fixation sur ces cellules de l'immunotoxine anti-T65.

La propriété fondamentale des immunotoxines étant d'inhiber la synthèse protéique des cellules-cibles, le test utilisé consiste à mesurer l'effet des substances étudiées sur l'incorporation de $^{14}C$-leucine dans les cellules cancéreuses en culture.

Cette mesure est effectuée selon une technique adaptée de celle décrite dans Journal of Biological Chemistry, 1974, 249 (11), 3557–3562 utilisant le traceur $^{14}C$-leucine pour la détermination du taux de synthèse protéique. La détermination de la radioactivité incorporée est effectuée ici sur les cellules entières isolées par filtration.

A partir de ces déterminations, on peut tracer les courbes effets/doses présentant en abscisse la concentration molaire en chaîne A des substances étudiées et en ordonnée l'incorporation de $^{14}C$-leucine exprimée en pourcentage de l'incorporation des cellules témoins en l'absence de toute substance affectant la synthèse protéique.

On peut ainsi déterminer pour chaque substance étudiée la concentration qui inhibe 50% de l'incorporation de $^{14}C$-leucine ou «concentration inhibitrice 50» (CI 50).

Les différents essais de cette expérience ont été conduits comme suit. Les résultats expérimentaux correspondants sont présentés dans la figure 1.

a) Des cellules CEM sont incubées 18 heures à 37 °C en présence de concentrations connues de ricine ou de chaîne A isolée, utilisées à titre de substances de référence, puis ces cellules sont soumises à l'étape d'incorporation du traceur radioactif. Les CI 50 obtenues sont respectivement $4 \cdot 10^{-12}$ M et $4,5 \cdot 10^{-8}$ M pour la ricine et la chaîne A. Il a été montré par ailleurs que ces valeurs sont indistinguables de celles que l'on obtient sur cellules CEM marquées par l'haptène TNP (courbe 1, ricine sur CEM et courbe 2 chaîne A ricine sur CEM).

b) Des cellules CEM marquées par le TNP sont incubées 18 heures à 37 °C en présence d'une immunotoxine à spécificité anti-DNP obtenue comme indiqué dans la demande précédente nº FR-A-2 437 213 et addition FR-A-2 466 252. puis soumises à l'étape d'incorporation du traceur radioactif. La forme de la courbe de cytotoxicité obtenue et la valeur de la CI 50 ($1,5 \cdot 10^{-9}$ M) montrent que ces cellules sont normalement sensibles à l'effet cytotoxique de l'immunotoxine anti-DNP, démontrant ainsi que le marquage par le TNP de ces cellules est correctement effectué (courbe 3).

c) Des cellules CEM marquées par le TNP sont d'abord incubées pendant 1 heure à 4 °C en présence d'uréase non conjuguée à raison de 4 U/ml, puis sont lavées, incubées 18 heures à 37 °C en présence de l'immunotoxine anti T65 et d'urée 5 mM et enfin soumises à l'étape d'incorporation du traceur radioactif. La CI 50 obtenue est

$5 \cdot 10^{-9}$ M. Cette valeur est identique à celle que l'on obtient en utilisant dans les mêmes conditions des cellules CEM non marquées au TNP, en l'absence de traitement par l'uréase et en l'absence d'urée dans le milieu d'incubation après lavage de l'uréase. Cet essai montre que l'incubation en présence d'uréase non conjuguée n'entraîne aucune liaison de l'uréase aux cellules et de ce fait aucune potentialisation de l'effet de l'immunotoxine anti T65 (courbe 4).

d) Des cellules CEM marquées par le TNP sont d'abord incubées pendant 1 heure à 4 °C en présence du conjugué immunoenzymatique décrit précédemment, utilisé à la concentration de 6,5 U/ml. Il a été vérifié par ailleurs que ce conjugué, employé dans ces conditions, ne présente aucune cytotoxicité propre sur les cellules utilisées. Ces cellules sont ensuite lavées pour éliminer tout conjugué qui ne serait pas fixé, puis elles sont incubées 18 heures à 37 °C en présence de l'immunotoxine anti T65 et d'urée 5 mM. Elles sont enfin soumises à l'étape d'incorporation du traceur radioactif. La CI 50 obtenue est $3,5 \cdot 10^{-13}$ M (courbe 5).

Ce résultat montre que l'effet potentialisateur du conjugué immunoenzymatique augmente d'environ 14 000 fois l'activité cytotoxique de l'immunotoxine sur les cellules-cibles. Cet essai démontre que cet effet potentialisateur implique la fixation du conjugué immunoenzymatique sur l'antigène correspondant à sa spécialité immunologique. Cette fixation résiste au lavage des cellules et laisse à la surface de celles-ci de l'uréase enzymatiquement active qui produit des ions $NH_4^+$ à partir de l'urée présente dans le milieu d'incubation avec l'immunotoxine, ce qui entraîne l'effet potentialisateur bien connu des ions $NH_4^+$. L'effet de potentialisation obtenu est tout à fait analogue à celui qui a été précédemment démontré par addition de chlorure d'ammonium 10 mM au milieu d'incubation.

Comme dans le cas d'une addition artificielle de chlorure d'ammonium, cet effet potentialisateur n'est obtenu ni avec la ricine, ni avec la chaîne A de ricine, ni avec une immunotixine non spécifique des cellules étudiées.

Dans les conditions de cet exemple, l'activité cytotoxique de l'immunotoxine anti T65, en présence du conjugué immunoenzymatique utilisé est environ 130 000 fois celle de la chaîne A de ricine et elle dépasse même en puissance celle de la ricine d'un facteur d'environ 11 fois.

Exemple 3

Conjugué immunoenzymatique obtenu par réaction entre un anticorps anti-dinitrophényle substitué par un groupement maléimide et une uréase d'origine végétale.

a) Anticorps anti-dinitrophényle (anti-DNP)

Cet anticorps est un anticorps monoclonal qui a été purifié par des techniques conventionnelles à partir du liquide d'ascite de souris de souche Balb/C auxquelles a été transplanté l'hybridome $F_9$.

Cet hybridome a lui-même été obtenu par fusion entre des cellules spléniques de souris de souche Balb/C immunisées à l'aide de γ-globuline bovine sur laquelle ont été préalablement fixés 20 radicaux DNP par mole avec des cellules de la lignée de myélome marin NS 1, et isolé par clonage, selon les techniques classiques. L'anticorps obtenu est une immunoglobuline de classe G et d'isotype 2b dont la constante d'affinité (mesurée pour le ligand ε-DNP-lysine) est de $1,8 \cdot 10^8$ $M^{-1}$.

b) Anticorps anti-DNP activé

A 2,5 ml d'anticorps anti-DNP à 9,7 mg/ml dans le tampon phosphate 125 mM pH 7,0 sont ajoutés 10 µl de diméthylformamide contenant 0,4 mg de l'ester N-hydroxysuccinimide de l'acide m-maléimidobenzoïque. Le mélange est incubé pendant ½ heure à 25 °C. La solution est ensuite déposée sur une colonne de Sephadex G 25 de 10 ml équilibrée dans le tampon phsphate 125 mM pH 7,0. L'élution est suivie par la mesure de la densité optique à 280 nm. On recueille 2,5 ml à partir du volume d'exclusion de la colonne. La mesure du taux de substitution est effectuée sur une aliquote par réaction avec un excès de $^{14}C$-cystéine.

On obtient ainsi une solution à 8 mg d'anticorps par ml, avec un taux de substitution de 3,5 groupements maléimide par mole d'anticorps.

c) Uréase

L'enzyme utilisée est l'uréase de provenance SIGMA (type VII, référence U 0376), titrant 170 unités par mg. Une unité est la quantité d'enzyme qui permet la libération de 1 micromole de $NH_4^+$ par minute à 20 °C et à pH 7,0, à partir d'urée.

Cette enzyme possède naturellement 27 groupements thiol par molécule de poids moléculaire 480 000. Ces groupements thiol, titrables par la méthode de ELLMAN, ne sont pas tous nécessaires à l'activité enzymatique. Certains peuvent donc servir à assurer le couplage avec l'anticorps activé.

d) Couplage de l'anticorps et de l'enzyme

Immédiatement après dessalage sur G 25, 2,4 ml de la solution d'anticorps activé sont mélangés à 2,0 ml de solution d'uréase à 24 mg/ml dans le tampon phosphate 125 mM pH 7,0. On incube le mélange 1 h à 25 °C et, après centrifugation, on dépose sur une colonne de 450 ml de gel Sephadex G 200® équilibrée dans le tampon PBS. L'élution est suivie par mesure de la densité optique à 280 nm et par mesure de l'activité uréase selon la technique de SUMMER.

On réunit les fractions contenant les plus fortes activités uréase et on obtient ainsi 14 ml de solution de conjugué à 102 unités par ml.

Si une fraction aliquote de cette solution est chromatographiée sur une colonne de gel de Proteine A-Sépharose®, on constate que 30% de l'activité uréase ne sont pas retenus sur la colonne. Le reste de l'activité uréase est élué en même temps que l'anticorps par le tampon de pH 3,5. Une expérience de contrôle démontre que l'uréase non couplée à l'anticorps n'est absolument pas fixée par la colonne. Ceci démontre que

70% de l'activité uréase des fractions réunies appartiennent bien à un conjugué anticorps-uréase. Pour les essais décrits ci-après, la solution n'a pas été débarrassée de l'uréase libre contaminante qui est sans effet dans les conditions employées, comme cela est décrit ci-après.

Exemple 4
Potentialisation de l'immunotoxine anti T65 par le conjugué immunoenzymatique de l'exemple 3.

Le conjugué selon l'invention, obtenu comme indiqué précédemment (exemple 3), a été étudié en ce qui concerne ses propriétés biologiques et plus spécialement sa capacité à potentialiser l'activité de l'immunotoxine anti T65 dans un modèle cellulaire approprié.

Ce modèle est constitué par des cellules de la lignée lymphoblastoïde humaine CEM qui portent naturellement l'antigène T65. Cet antigène, contre lequel est dirigée l'immunotoxine utilisée, constitue le premier antigène-cible du modèle. On peut, en outre, marquer ces cellules à l'aide de l'haptène trinitrophényle (TNP) selon la technique décrite dans notre demande antérieure n° FR-A-2 437 213. Cet haptène est très bien reconnu par l'anticorps anti-DNP contenu dans le conjugué immunoenzymatique étudié et constitue donc le deuxième antigène-cible du modèle. Il a été démontré que le marquage des cellules par l'haptène TNP ne modifie pas la viabilité des cellules et ne perturbe pas la fixation sur ces cellules de l'immunotoxine anti T65.

La propriété fondamentale des immunotoxines étant d'inhiber la synthèse protéique des cellules-cibles, le test utilisé consiste à mesurer l'effet des substances étudiées sur l'incorporation de $^{14}C$-leucine dans les cellules cancéreuses en culture.

Cette mesure est effectuée selon une technique adaptée de celle décrite dans Journal of Biological Chemistry, 1974, 249 (11), 3557–62 utilisant le traceur $^{14}C$-leucine pour la détermination du taux de synthèse protéique. La détermination de la radioactivité incorporée est effectuée ici sur les cellules entières isolées par filtration.

A partir de ces déterminations, on peut tracer les courbes effets/doses présentant en abscisse la concentration molaire en chaîne A des substances étudiées et en ordonnée l'incorporation de $^{14}C$-leucine exprimée en pourcentage de l'incorporation des cellules témoins en l'absence de toute substance affectant la synthèse protéique.

On peut ainsi déterminer pour chaque substance étudiée la concentration qui inhibe 50% de l'incorporation de $^{14}C$-leucine ou «concentration inhibitrice 50» (CI 50).

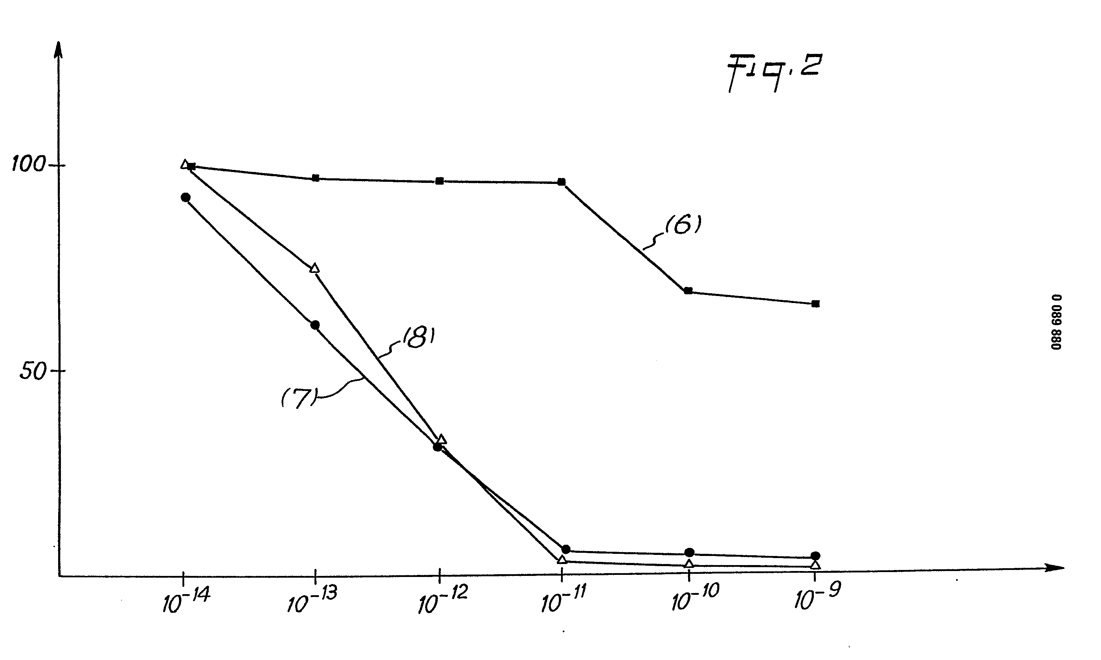

Les différents essais de cette expérience ont été conduits comme suit. Les résultats expérimentaux correspondants sont présentés dans la figure 2.

a) Les essais témoins effectués à l'exemple 2 a) n'ont pas été répétés puisqu'ils peuvent être considérés comme valables dans le présent exemple.

b) Des cellules CEM marquées par le TNP sont incubées 18 h à 37 °C en présence d'une immunotoxine à spécificité anti T65 obtenue comme indiqué dans la demande précédente n° FR-A-2 516 794, puis soumises à l'étape d'incorporation du traceur radioactif. La forme de la courbe de cytotoxicité est identique à celle que l'on obtient avec les mêmes cellules non marquées au TNP dans les mêmes conditions d'incubation, démontrant ainsi que le marquage par le TNP de ces cellules est correctement effectué (courbe 6).

c) Des cellules CEM marquées par le TNP sont d'abord incubées pendant 1 h à 4 °C en présence d'uréase non conjuguée à raison de 1 U/ml, puis sont lavées, incubées 18 h à 37 °C en présence de l'immunotoxine anti T 65 à la seule concentration de $10^{-9}$ M et d'urée 5 mM et enfin soumises à l'étape d'incorporation du traceur radioactif.

La valeur d'incorporation de $^{14}C$-leucine obtenue dans cet essai (65%) est indistinguable de celle obtenue par la même concentration d'immunotoxine anti T65 dans l'essai b). Ce résultat montre que l'incubation en présence d'uréase non conjuguée n'entraîne aucune liaison de l'uréase aux cellules et, de ce fait, aucune potentialisation de l'effet de l'immunotoxine anti T65.

d) Des cellules CEM marquées par le TNP sont d'abord incubées pendant 1 h à 4 °C en présence du conjugué immunoenzymatique décrit précédemment, utilisé à la concentration de 1,02 U/ml. Il a été vérifié par ailleurs que ce conjugué, employé dans ces conditions, ne présente aucune cytotoxicité propre sur les cellules utilisées. Ces cellules sont ensuite lavées pour éliminer tout conjugué qui ne seait pas fixé, puis elles sont incubées 18 h à 37 °C en présence de l'immunotoxine anti T65 et d'urée 5 mM. Elles sont enfin soumises à l'étape d'incorporation du traceur radioactif. La CI 50 obtenue est $2,4 \cdot 10^{-13}$ M (courbe 7).

Cette valeur tout à fait comparable à celle obtenue en utilisant le conjugué de l'exemple 1, représente un effet potentialisateur remarquable exercé par le conjugué immunoenzymatique à l'égard de l'immunotoxine.

Cet essai démontre que cet effet potentialisateur implique la fixation du conjugué immunoenzymatique sur l'antigène correspondant à sa spécificité immunologique. Cette fixation résisite au lavage des cellules et laisse à la surface de celles-ci de l'uréase enzymatiquement active qui produit des ions $NH_4^+$ à partir de l'urée présente dans le milieu d'incubation avec l'immunotoxine, ce qui entraîne l'effet potentialisateur des ions $NH_4^+$.

L'effet de potentialisation obtenu est tout à fait analogue à celui qui est démontré lorsque l'incubation a lieu en présence de chlorure d'ammonium 10 mM ajouté au milieu d'incubation à la place du système conjugué immunoenzymatique/substrat.

Dans le cas d'addition de chlorure d'ammonium, la CI 50 obtenue est en effet $3,8 \times 10^{-13}$ M comme le montre la courbe correspondante de la figure 2 (courbe 8).

Les exemples ci-dessus montrent que les produits selon l'invention peuvent être utilisés en thérapeutique humaine.

Les nouveaux médicaments selon l'invention sont conditionnés pour être utilisés par voie injectable et de préférence par voie intraveineuse. Ils peuvent être utilisés pour le traitement des affections, cancéreuses ou non, qui seraient sensibles à l'anticorps utilisé pour la préparation de l'immunotoxine. Ils seront employés à des doses et dans des conditions qu'il conviendra de déterminer dans chaque cas en fonction du sujet et de la nature de l'affection à traiter.

**Revendications pour les Etats contractants: DE, NL, GB, CH, FR, LU, BE,SE, IT**

1. Conjugués immunoenzymatiques pour utilisation comme potentialisateurs d'immunotoxines dans une association médicamenteuse contenant une immunotoxine obtenue par couplage, par liaison covalente, de la chaîne A de la ricine avec des anticorps ou fragments d'anticorps dirigés contre un antigène porté par une cellule cible, caractérisés en ce qu'ils résultent du couplage chimique par liaison covalente, entre un anticorps ou un fragment d'anticorps ayant conservé la capacité de reconnaître un antigène porté par la même cellule-cible et une enzyme capable de libérer des ions ammonium à partir de substrats naturels bien tolérés dans les organismes animaux supérieurs.

2. Conjugués selon la revendication 1, caractérisés en ce que la liaison covalente est une liaison disulfure.

3. Conjugués selon la revendication 1 caractérisés en ce que la liaison covalente est une liaison thioéther.

4. Procédé pour la préparation de conjugués selon l'une des revendications 1 ou 2, caractérisés en ce que l'on réalise la réaction d'une protéine possédant une fonction thiol soit $P_1SH$ avec une autre protéine $P_2$ dans laquelle aura été introduite au moins la fonction comportant un pont disulfure et un radical réagissant avec le thiol, soit $P_2$–S–S–X, ladite réaction étant effectuée en milieu aqueux, à pH compris entre 5 et 9 et à une température inférieure à environ 30 °C, les protéines $P_1$ et $P_2$ étant l'une l'anticorps et l'autre l'enzyme.

5. Procédé pour la préparation de conjugués selon l'une des revendications 1 ou 3, caractérisé en ce qu'on fait réagir une protéine possédant une fonction thiol, à savoir $P_1SH$ avec une autre protéine $P_2$ dans laquelle aura été introduite la fonction maléimide à savoir

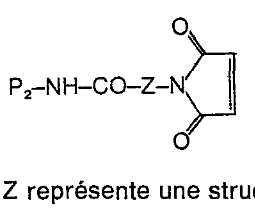

dans laquelle Z représente une structure d'espacement, la réaction étant effectuée en milieu aqueux, à une température inférieure à environ 30 °C les protéines $P_1$ et $P_2$ étant l'une l'anticorps et l'autre l'enzyme.

6. Association médicamenteuse caractérisée en ce qu'elle comporte au moins une immunotoxine obtenue par couplage, par liaison covalente de la chaîne A de la ricine avec des anticorps ou fragments d'anticorps dirigés contre un antigène porté par une cellule-cible et au moins un conjugué immunoenzymatique selon l'une des revendications 1 à 3.

7. Association selon la revendication 6, caractérisée en ce qu'elle est conditionnée de façon à être utilisée par voie injectable de préférence par voie veineuse.

**Revendications pour l'Etat contractant AT**

1. Procédé pour l'obtention de conjugués immunoenzymatiques utiles comme potentialisateurs d'immunotoxines dans une association médicamenteuse contenant une immunotoxine obtenue par couplage, par liaison covalente, de la chaîne A de la ricine avec des anticorps ou fragments d'anticorps dirigés contre un antigène porté par une cellule-cible, caractérisé en ce qu'il consiste à coupler chimiquement, par liaison covalente, un anticorps ou un fragment d'anticorps ayant conservé la capacité de reconnaître un antigène porté par la même cellule-cible et une enzyme capable de libérer des ions ammonium à partir de substrats naturels bien tolérés dans les organismes animaux supérieurs.

2. Procédé selon la revendication 1, caractérisé en ce que la liaison covalente est une liaison disulfure.

3. Procédé selon la revendication 1, caractérisé en ce que la liaison covalente est une liaison thioéther.

4. Procédé selon l'une des revendications 1 ou 2, caractérisé en ce que l'on réalise la réaction d'une protéine possédant une fonction thiol soit $P_1SH$, avec une autre protéine $P_2$ dans laquelle aura été introduite au moins la fonction comportant un pont disulfure et un radical réagissant avec le thiol, soit $P_2$–S–S–X, ladite réaction étant effectuée en milieux aqueux, à pH compris entre 5 et 9 et à température inférieure à environ 30 °C, les protéines $P_1$ et $P_2$ étant l'une l'anticorps et l'autre l'enzyme.

5. Procédé selon l'une des revendications 1 ou 3, caractérisé en ce qu'on fait réagir une protéine possédant une fonction thiol, à savoir $P_1$ SH, avec une autre protéine $P_2$ dans laquelle aura été introduite la fonction maléimide, à savoir

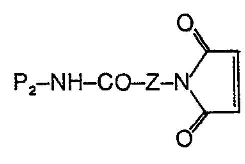

dans laquelle Z représente une structure d'espacement, la réaction étant effectuée en milieu aqueux, à une température inférieure à environ 30 °C, les protéines $P_1$ et $P_2$ étant l'une l'anticorps et l'autre l'enzyme.

6. Utilisation des conjugués immunoenzymatiques obtenus selon l'une quelconque des revendications 1 à 3, pour la préparation d'associations médicamenteuses comportant au moins une immunotoxine obtenue par couplage, par liaison covalente, de la chaîne A de la ricine avec des anticorps ou fragments d'anticorps dirigés contre

un antigène porté par une cellule-cible et au moins un conjugué immunoenzymatique.

7. Utilisation selon la revendication 6, caractérisée en ce que l'association médicamenteuse est conditionnée de façon à être utilisée par voie injectable et de préférence par voie veineuse.

### Claims for the contracting states: DE, NL, GB, CH, FR, LU, BE, SE, IT, LI

1. Immunoenzymic conjugates for the use as potentiators of immunotoxins in a medicinal association containing an immunotoxin obtained by coupling by covalent bond, of chain A of ricin with antibodies or fragments of antibodies directed against an antigen carried by a target cell, characterized in that they result from a chemical coupling by covalent bond, of an antibody or a fragment of antibody having retained the capacity to recognize an antigen carried by the same target cell with an enzyme capable of liberating ammonium ions from natural substrates well tolerated in higher animal organisms.

2. Conjugates according to claim 1, characterized in that the covalent bond is a disulfide bond.

3. Conjugates according to claim 1, characterized in that the covalent bond is a thioether bond.

4. Process for preparing the conjugates according to any one of claims 1 or 2, characterized in that a protein having a thiol function, namely $P_1SH$, is reacted with another protein $P_2$ in which at least the function comprising a disulfide bridge and a radical acting with thiol, namely $P_2$–S–S–X, has been introduced beforehand, said reaction being conducted in aqueous medium, at a pH between 5 and 9 and at a temperature less that an about 30 °C, the proteins $P_1$ and $P_2$ being one the antibody and the other the enzyme.

5. Process for preparing the conjugates according to any one of claims 1 or 3, characterized in that a protein having a thiol function, namely $P_1SH$, is reacted with another protein $P_2$ in which the maleimide function has been introduced beforehand, namely

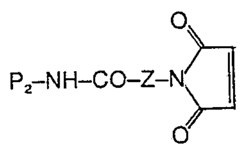

in which Z is a spacing structure, the reaction being conducted in aqueous medium, at a temperature less than about 30 °C, the proteins $P_1$ and $P_2$ being one the antibody and the other the enzyme.

6. Medicinal association characterized in that it contains at least one immunotoxin obtained by coupling by covalent bond, of chain A of ricin with antibodies or fragments of antibodies directed against an antigen carried by a target cell and at least one immunoenzymic conjugate according to any one of claims 1 to 3.

7. Association according to claim 6, characterized in that it is presented so as to be used by injectable route, preferably by veinous route.

### Claims for the contracting state AT

1. Process for obtaining immunoenzymic conjugates useful as potentiators of immunotoxins in a medicinal association containing an immunotoxin obtained by coupling by covalent bond, of chain A of ricin with antibodies or fragments of antibodies directed against an antigen carried by a target cell, characterized in that it consists in chemically coupling by covalent bond an antibody or a fragment of antibody having retained the capacity to recognize an antigen carried by the same target cell with an enzyme capable of liberating ammonium ions from natural substrates well tolerated in higher animal organisms.

2. Process according to claim 1, characterized in that the covalent bond is a disulfide bond.

3. Process according to claim 1, characterized in that the covalent bond is a thioether bond.

4. Process according to any one of claims 1 or 2, characterized in that a protein having a thiol function, namely $P_1SH$, is reacted with another protein $P_2$ in which at least the function comprising a disulfide bridge and a radical acting with thiol, namely $P_2$–S–S–X, has been introduced beforehand, said reaction being conducted in aqueous medium, at a pH between 5 and 9 and at a temperature less than about 30 °C, the proteins $P_1$ and $P_2$ being one the antibody and the other the enzyme.

5. Process according to any one of claims 1 or 3, characterized in that a protein having a thiol function, namely $P_1SH$, is reacted with another protein $P_2$ in which the maleimide function has been introduced beforehand, namely

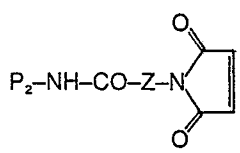

in which Z is a spacing structure, the reaction being conducted in aqueous medium, at a temperature less than about 30 °C, the proteins $P_1$ and $P_2$ being one the antibody and the other the enzyme.

6. Use of immunoenzymic conjugates obtained according to any one of claims 1 to 3 for the preparation of medicinal associations containing at least one immunotoxin obtained by coupling by covalent bond, of chain A of ricin with antibodies or fragments of antibodies directed against an antigen carried by a target cell and at least one immunoenzymic conjugate.

7. Use according to claim 6, characterized in that the medicinal association is presented so as to be used by injectable route and preferably by veinous route.

**Patentansprüche für die Vertragsstaaten DE, NL, GB, CH, FR, LU, BE, SE, IT, LI**

1. Immunenzymatische Konjugate zur Verwendung als Potentiatoren von Antitoxinen in einer Medikamentenkombination, die ein durch Kopplung mittels kovalenter Bindung der Kette A von Ricin mit Antikörpern oder Antikörperfragmenten, die gegen ein von einer Zielzelle getragenes Antigen gerichtet sind, erhaltenes Antitoxin enthält, dadurch gekennzeichnet, dass sie aus der chemischen Kopplung mittels kovalenter Bindung zwischen einem Antikörpoer oder Antikörperfragment, das die Fähigkeit, ein von derselben Zielzelle getragenes Antigen zu erkennen, bewahrt hat, und einem Enzym, das aus natürlichen, von den Organismen höherer Lebewesen gut verträglichen Substraten Ammoniumionen freisetzen kann, resultieren.

2. Konjugate nach Anspruch 1, dadurch gekennzeichnet, dass die kovalente Bindung eine Disulfidbindung ist.

3. Konjugate nach Anspruch 1, dadurch gekennzeichnet, dass die kovalente Bindung eine Thioätherbindung ist.

4. Verfahren zur Herstellung von Konjugaten nach einem der Ansprüche 1 oder 2, dadurch gekennzeichnet, dass man die Umsetzung eines eine Thiolfunktion aufweisenden Proteins, nämlich $P_1SH$, mit einem anderen Protein $P_2$, in welches wenigstens die eine Disulfidbrücke umfassende Funktion und ein mit dem Thiol reagierendes Radikal eingeführt worden ist, nämlich $P_2$–S–S–SX, vorgenommen wird, welche Reaktion in wässerigem Milieu bei einem pH zwischen 5 und 9 und einer Temperatur von weniger als etwa 30 °C durchgeführt wird, wobei die Proteine $P_1$ und $P_2$ das eine der Antikörper und das andere das Enzym sind.

5. Verfahren zur Herstellung von Konjugaten nach einem der Ansprüche 1 oder 3, dadurch gekennzeichnet, dass man ein eine Thiolfunktion aufweisendes Protein, nämlich $P_1SH$, mit einem anderen Protein $P_2$, in das die Maleinimidfunktion eingeführt worden ist, nämlich

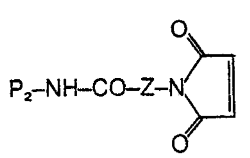

worin Z eine Abstandhalterstruktur darstellt, reagieren lässt, welche Reaktion in wässerigem Milieu bei einer Temperatur von weniger als etwa 30 °C durchgeführt wird, wobei die Proteine $P_1$ und $P_2$ das eine der Antikörper und das andere das Enzym sind.

6. Medikamentenkombination, dadurch gekennzeichnet, dass sie mindestens ein durch Kopplung mittels kovalenter Bindung der Kette A von Ricin mit Antikörpern oder Antikörperfragmenten, die gegen ein von einer Zielzelle getragenes Antigen gerichtet sind, erhaltenes Antitoxin und mindestens ein immunenzymatisches Konjugat nach einem der Ansprüche 1 bis 3 enthalten.

7. Kombination nach Anspruch 6, dadurch gekennzeichnet, dass sie zur Verwendung auf Injektionsweg, vorzugsweise venösem Weg, konditioniert sind.

**Patentansprüche für den Vertragsstaat AT**

1. Verfahren zur Herstellung von immunenzymatischen Konjugaten, die als Potentiatoren von Antitoxinen in einer Medikamentenkombination, die ein durch Kopplung mittels kovalenter Bindung der Kette A von Ricin mit Antikörpern oder Antikörperfragmenten, die gegen ein von einer Zielzelle getragenes Antigen gerichtet sind, erhaltenes Antitoxin enthält, nützlich sind, dadurch gekennzeichnet, dass es in der chemischen Kopplung mittels kovalenter Bindung zwischen einem Antikörper oder Antikörperfragment, das die Fähigkeit, ein von derselben Zielzelle getragenes Antigen zu erkennen, bewahrt hat, und einem Enzym, das aus natürlichen, von den Organismen höherer Lebewesen gut verträglichen Substraten Ammoniumionen freisetzen kann, besteht.

2. Verfahren nach Anspruch 1, dadurch gekennzeichnet, dass die kovalente Bindung eine Disulfidbindung ist.

3. Verfahren nach Anspruch 1, dadurch gekennzeichnet, dass die kovalente Bindung eine Thioätherbindung ist.

4. Verfahren nach einem der Ansprüche 1 oder 2, dadurch gekennzeichnet, dass man die Umsetzung eines eine Thiolfunktion aufweisenden Proteins, nämlich $P_1SH$, mit einem anderen Protein $P_2$, in welches wenigstens die eine Disulfidbrücke umfassende Funktion und ein mit dem Thiol reagierendes Radikal eingeführt worden ist, nämlich $P_2$–S–S–X, vorgenommen wird, welche Reaktion in wässerigem Milieu bei einem pH zwischen 5 und 9 und einer Temperatur von weniger als etwa 30 °C durchgeführt wird, wobei die Proteine $P_1$ und $P_2$ das eine der Antikörper und das andere das Enzym sind.

5. Verfahren nach einem der Ansprüche 1 oder 3, dadurch gekennzeichnet, dass man ein eine Thiolfunktion aufweisendes Protein, nämlich $P_1SH$, mit einem anderenm Protein $P_2$, in das die Maleinimidfunktion eingeführt worden ist, nämlich

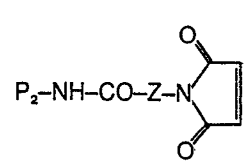

worin Z eine Abstandhalterstruktur darstellt, reagieren lässt, welche Reaktion in wässerigem Milieu bei einer Temperatur von weniger als etwa 30 °C durchgeführt wird, wobei die Proteine $P_1$ und $P_2$ das eine der Antikörper und das andere das Enzym sind.

6. Verwendung der nach einem der Ansprüche 1 bis 3 erhaltenen immunenzymatischen Konjugate zur Herstellung von Medikamentenkombinationen, die mindestens ein durch Kopplung mittels kovalenter Bindung der Kette A von Ricin mit Antikörpern oder Antikörperfragmenten, die gegen ein von einer Zielzelle getragenes Antigen gerichtet sind, erhaltenes Antitoxin und mindestens ein immunenzymatisches Konjugat umfassen.

7. Verwendung nach Anspruch 6, dadurch gekennzeichnet, dass die Medikamentenkombination zur Verwendung auf Injektionsweg, vorzugsweise venösem Weg, konditioniert sind.

Fig.1

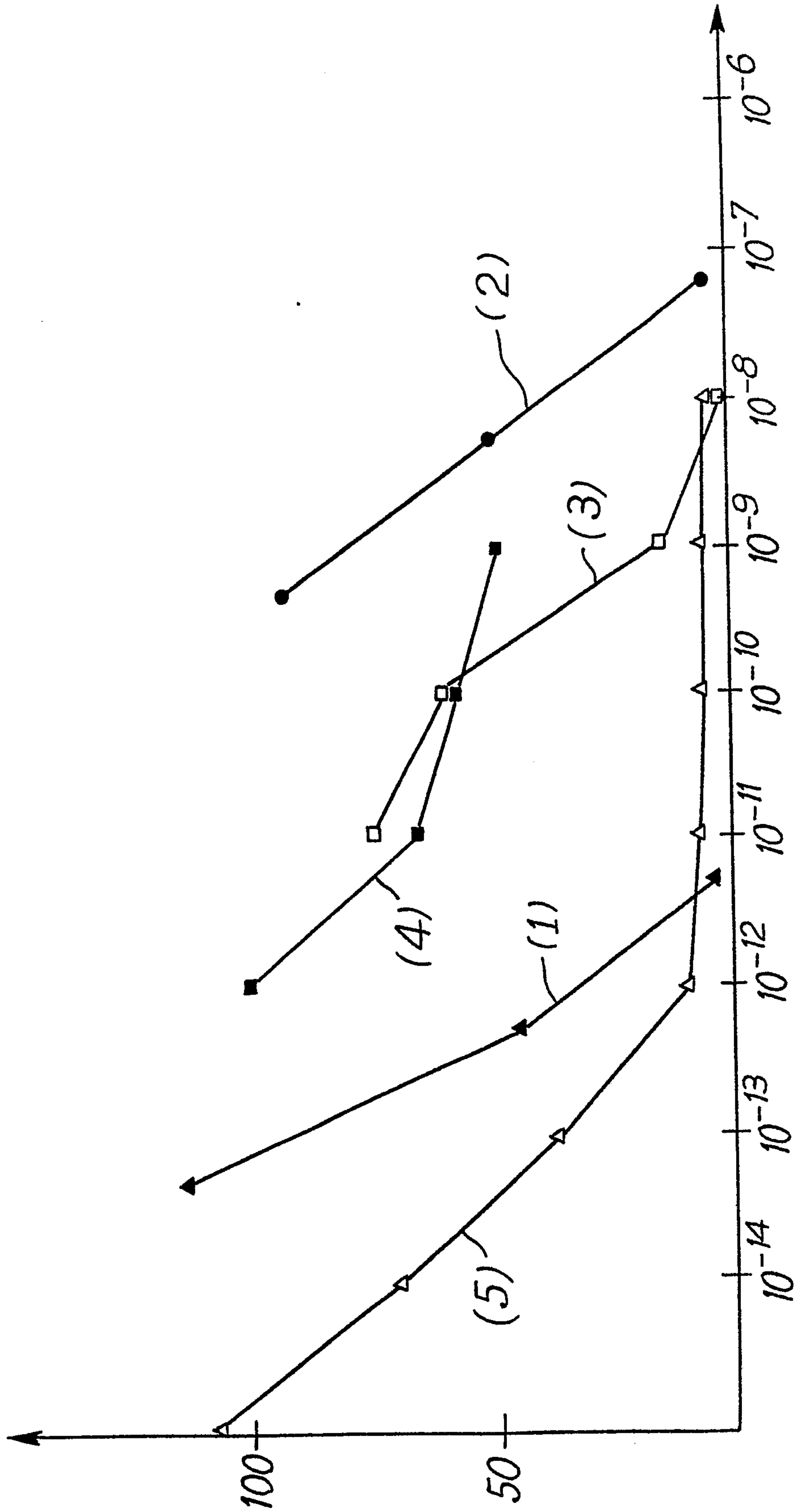

Fig. 2